# EUROPÄISCHE PATENTANMELDUNG

(11) **EP 4 755 302 A1**
(43) Veröffentlichungstag der Anmeldung: **10.06.2026**
(21) Anmeldenummer: 25221960.5
(22) Anmeldetag: 09.12.2025
(51) Int. Cl.: A61B 5/296, A61B 5/389, A61B 5/00

(54) **VORRICHTUNG UND SIGNALVERARBEITUNGSEINHEIT ZUM TRAINIEREN DER BECKENBODENMUSKULATUR**

(30) Priorität: 09.12.2024 DE 202024107133 U
(71) Anmelder: Fysor GmbH, 71093 Weil im Schönbuch (DE)
(72) Erfinder: Kässer, Mark, 71093 Weil im Schönbuch (DE); Möller, Andreas, 72072 Tübingen (DE); Hämmerle, Ingo, 70569 Stuttgart (DE); Helmhold, Florian, 72070 Tübingen (DE)
(74) Vertreter: Schieler, Paul Manfred Robert

(57) **Zusammenfassung**

Diese Anmeldung betrifft eine Vorrichtung zur Auswertung einer Muskelaktivität einer Beckenbodenmuskulatur eines Beckenbodens, wobei die Vorrichtung eine Signalverarbeitungseinheit und eine extrakorporale Elektrodeneinheit aufweist, wobei die Elektrodeneinheit eine Messelektrode und eine Referenzelektrode aufweist, wobei die Elektrodeneinheit ausgelegt ist, ein Elektromyographiesignal zu messen.

## Beschreibung

Die Erfindung geht aus von einer Vorrichtung oder einer Signalverarbeitungseinheit zum Trainieren der menschlichen Beckenbodenmuskulatur nach Gattung der unabhängigen Ansprüche.

Aus der KR20140103694A ist bereits eine Vorrichtung zur Stärkung der Beckenbodenmuskulatur eines Anwenders mit Verwendung von EMG, Elektromyographie, bekannt, welche einen EMG-Sensor in einem Sitzkissen verwendet, dessen Signale analysiert werden, um den Anwender beim Training der Beckenbodenmuskulatur anzuleiten.

Aus der US9993688B2 ist bereits eine Vorrichtung und ein Verfahren zum Anweisen und zum Erfassen von Beckenübungen bekannt, welche einen Sensor verwenden, der in ein flexibles Polymermaterial eingebettet ist, teilweise innerhalb und teilweise außerhalb des Körpers appliziert wird und eine Muskelkraft misst.

Ein erster Aspekt der Erfindung betrifft eine Vorrichtung zur Auswertung einer Muskelaktivität einer Beckenbodenmuskulatur mit einer Signalverarbeitungseinheit und einer extrakorporalen Elektrodeneinheit, welche eine Messelektrode und eine Referenzelektrode aufweist, wobei die Elektrodeneinheit ausgelegt ist, ein Elektromyographiesignal zu messen. Der Abstand zwischen der Messelektrode und der Referenzelektrode beträgt vorzugsweise 10 mm bis 100 mm, bevorzugter 15 mm bis 80 mm, besonders bevorzugt 20 mm bis 70 mm am bevorzugsten 30 mm bis 50 mm oder zwischen 30 mm und 60 mm oder zwischen 40 mm und 60 mm.

Dies hat gegenüber dem Stand der Technik den Vorteil, dass eine Messung eines Oberflächenelektromyographiesignals auf der Haut in der Gesäßfalte eines Anwenders, d.h. extrakorporal und nicht-invasiv, erfolgen kann.

Die Signalverarbeitungseinheit kann als Signalverarbeitungseinheit im Sinne der nachfolgend beschriebenen Signalverarbeitungseinheit gemäß dem zweiten Aspekt der Erfindung und/oder deren Ausführungsformen ausgebildet sein.

Ein zweiter Aspekt der Erfindung betrifft eine Signalverarbeitungseinheit, zur Auswertung einer Aktivität einer Beckenbodenmuskulatur über ein Elektromyographiesignal einer extrakorporal auf das Steißbein angelegten Elektrodeneinheit, die dazu ausgelegt ist das Elektromyographiesignal zu filtern, das gefilterte Elektromyographiesignal zu kalibrieren eine Kontraktion und/oder einer Kontraktionsstärke der Beckenbodenmuskulatur anhand des gefilterten und kalibrierten Elektromyographiesignals zu ermitteln, und die Kontraktion zurückzumelden, insbesondere in Form eines Biofeedbacks an einen Anwender, insbesondere an die Person deren Beckenbodenaktivität ausgewertet wird.

Dies hat den Vorteil, dass vollautomatisch ein personenindividuelles Maß für eine Anstrengung der Aktivität der Beckenbodenmuskulatur ermittelt wird, das sich zur Steuerung von Videospielen eignet. Darüber hinaus kompensiert das Filtern bei Ausführungsformen (insbesondere wie weiter unten beschrieben) ohne Erdung, mit Erdung aber ohne separate Erdungselektrode und mit separater Erdungselektrode, die jedoch am gleichen Elektrodenträger angebracht ist wie eine Messelektrode und eine Referenzelektrode, die gegenüber Ausführungsformen, bei der die Erdung beispielsweise beabstandet von dem Elektrodenträger an der Hüfte angebracht wird, höhere Störanfälligkeit des gemessenen Elektromyographiesignals.

Durch die in den abhängigen Ansprüchen aufgeführten Maßnahmen sind vorteilhafte Weiterbildungen und Verbesserungen der in den unabhängigen Ansprüchen angegebenen Vorrichtung möglich.

Gemäß einer Ausführungsform ist die Elektrodeneinheit datenübertragungsgemäß, insbesondere mittels einer elektrischen Leitung, mit der Signalverarbeitungseinheit verbunden, insbesondere um das gemessene Elektromyographiesignal von der Elektrodeneinheit an die Signalverarbeitungseinheit zu übertragen. Vorzugsweise erfolgt die datenübertragungsgemäße Verbindung über eine elektrische Leitung, besonders bevorzugt über ein Kabel, das eine elektrische Leitung und einen die elektrische Leitung isolierenden Mantel aufweist. In einer Variante dieser Ausführungsform kann die elektrische Leitung, insbesondre das Kabel, mindestens 10 cm, 20 cm, 30 cm, 40 cm, 50 cm, 60 cm, 70 cm, 80 cm oder 90 cm lang, sein, um eine Anbringung der Elektrodeneinheit und der Signalverarbeitungseinheit an unterschiedlichen Körperstellen positionieren zu können. In einer alternativen Variante kann die elektrische Leitung, insbesondere das Kabel, kleiner als 10 cm, 8 cm, 5 cm oder 3 cm sein und/oder vollständig mit der Signalverarbeitungseinheit in einem Elektrodenträger der Elektrodeneinheit angeordnet sein. Die Elektroden haben vorzugsweise einen Durchmesser zwischen 3 mm und 35 mm, bevorzugter zwischen 5 mm und 30 mm, besonders bevorzugt zwischen 8 mm und 25 mm, am bevorzugsten zwischen 10 mm und 20 mm. Dabei bezieht sich der Durchmesser insbesondere auf den größten Durchmesser der Elektrode, vorzugsweise auf den Durchmesser der auf die Haut aufzulegende Fläche der Elektrode, beispielsweise auf die Mess- und Klebefläche der Elektrode. Vorzugsweise sind die Elektroden Ag/AgCl-Elektroden.

Gemäß einer weiteren Ausführungsform, die mit der vorherigen Ausführungsform kombiniert werden kann, weist die Elektrodeneinheit einen Elektrodenträger auf in und/oder auf dem die Messelektrode und die Referenzelektrode angeordnet, insbesondere befestigt, sind. Der Elektrodenträger kann aus einem Trägermaterial, vorzugsweise aus hautverträglichem Trägermaterial, besonders bevorzugt aus einem Kunststoff, insbesondere aus Silikon, Nylon, Polyethylenterephthalat, Acrylnitril-Butadien-Styrol-Copolymer oder aus Polyurethane, gebildet sein. Vorteilhaft an diesen Materialien ist, dass sie dem Elektrodenträger, insbesondere in Kombination mit der nachfolgend beschriebenen bevorzugten Dimensionierung, eine ausreichende Steifigkeit verleihen können, um die Elektroden in einem definierten Abstand zueinander zu halten und entlang der Gesäßfalte eines Anwenders zu positionieren. Der Elektrodenträger kann mittels 3D-Druck, insbesondere aus Nylon, oder mittels Spritzgusses, insbesondere aus Polyethylenterephthalat, Acrylnitril-Butadien-Styrol-Copolymer oder aus Polyurethane, hergestellt werde. Durch den Einsatz eines Elektrodenträgers wird die Anbringung der Elektroden an der richtigen Position gegenüber dem Anbringen von Einzelelektroden vereinfacht. Vorzugsweise ist der Elektrodenträger, länglich, insbesondere stab- oder knochenförmig, ausgebildet. Unter länglich ist insbesondere zu verstehen, dass der Elektrodenträger in einer Richtung, insbesondere in Längsrichtung, wenigstens 1,5 mal, 2 mal, 2,5, 3 mal oder 3,5 mal so groß ist, wie in Breiten- und Tiefenrichtung, insbesondere je nachdem welche Richtung die größere Erstreckung aufweist, was vorzugsweise die Breitenrichtung ist. Beispielsweise ist der Elektrodenträger in Längsrichtung vorzugsweise ca. 3,6 mal so groß wie in Breitenrichtung. Durch die längliche Ausgestaltung des Elektrodenträgers kann die Positionierung abermals vereinfacht werden, weil die Gesäßfalte des Anwenders bei länglichen Trägern eine Zentrierfunktion bereitstellt. Vorzugsweise sind Messelektrode und die Referenzelektrode an in Längsrichtung gegenüberliegenden Seiten des Elektrodenträgers angeordnet. Dadurch wird sichergestellt, dass die Messelektrode und die Referenzelektrode entlang der Gesäßfalte ausgerichtet werden. Vorzugsweise sind die Messelektrode und die Referenzelektrode an den Enden des Elektrodenträgers angeordnet, wobei der Elektrodenträger sich zwischen Messelektrode und Referenzelektrode verjüngt, insbesondere kontinuierlich und/oder knochenförmig verjüngt, wodurch eine stabile Anbringung des Elektrodenträgers in den nachfolgend beschriebenen bevorzugten Positionen ermöglicht wird. Vorzugsweise ist der Elektrodenträger dazu ausgebildet, zwischen Anus und Kreuzbein/unterem Rücken, vorzugsweise auf dem Steiß- und/oder Kreuzbein, eines Anwenders, dessen Muskelaktivität ausgewertet werden soll, angeordnet zu werden, wobei die Elektrodeneinheit besonders bevorzugt dazu ausgebildet ist, so zwischen Anus und Steißbein positioniert zu werden, dass die Messelektrode, zwischen 0,5 cm und 2 cm oberhalb des Anus angeordnet ist.

Vorzugsweise ist die Vorrichtung dazu ausgebildet, den Elektrodenträger entlang der Gesäßfalte an einer beliebigen Position anordnen zu können. Hierzu kann der Elektrodenträger in einer ersten Variante relativ zum Rest der Vorrichtung beweglich ausgebildet sein. Dabei kann der Rest der Vorrichtung die Signalverarbeitungseinheit und eine diese mit dem Elektrodenträger verbindende elektrisch Leitung, vorzugsweise Kabel, aufweisen. Vorzugsweise weist die Vorrichtung darüber hinaus keine mit dem Elektrodenträger verbundenen Bestandteile auf. Vorzugsweise ist die elektrische Leitung, insbesondere das Kabel, wenigstens 10 cm, 20 cm, 30 cm, 40 cm, 50 cm, 60 cm, 70 cm, 80 cm, 90 cm oder 100 cm lang, um die Signalverarbeitungseinheit weit genug weg von dem Elektrodenträger positionieren zu können, sodass der Elektrodenträger frei entlang der Gesäßfalte positioniert werden kann. Vorzugsweise ist die Vorrichtung zwischen dem Elektrodenträger und der Signalverarbeitungseinheit, insbesondere abgesehen von einem diese miteinander verbindenden Kabel, frei von weiteren Komponenten, insbesondere von Einlagen und/oder weiteren Sensoren, wie Feuchtigkeitssensoren. In einer alternativen Variante kann die Signalverarbeitungseinheit in dem Elektrodenträger integriert sein, sodass der Elektrodenträger zusammen mit der Signalverarbeitungseinheit frei entlang der Gesäßfalte positioniert werden kann. Des Weiteren kann die freie Positionierbarkeit des Elektrodenträgers, insbesondere für beide der zuvor definierten Varianten, durch die nachfolgend beschriebene Dimensionierung, insbesondere längliche Form, des Elektrodenträgers gewährleistet werden. Vorzugsweise erstreckt sich der Elektrodenträger in Längsrichtung um 30 mm bis 70 mm, vorzugsweise um 40 mm bis 60 mm, besonders bevorzugt um 45 mm bis 50 mm, beispielsweise um 47 mm, und/oder in Breitenrichtung an der breitesten Stelle um 5 mm bis 30 mm, vorzugsweise um 8 mm bis 20 mm, besonders bevorzugt um 10 mm bis 15 mm, beispielsweise um 13 mm, und/oder in Tiefenrichtung um 2 mm bis 20 mm, vorzugsweise um 3 mm bis 15 mm, besonders bevorzugt um 4 mm bis 8 mm, beispielsweise um 6 mm.

Vorzugsweise ist die Vorrichtung als Ganzes dazu ausgebildet unabhängig von der Kleidung, insbesondere der Unterwäsche, eines Anwenders, frei an dessen Körper angebracht zu werden, insbesondere entlang der Gesäßfalte angebracht zu werden. Dafür ist die Vorrichtung vorzugsweise frei von mit der Kleidung fest verbundenen oder in diese einlegbaren Komponenten, wie Flüssigkeit aufnehmende Materialien, insbesondere Einlagen, und/oder Flüssigkeitssensoren, insbesondere frei von Sensoren, die nicht für die Messung von Elektromyographiesignale verantwortlich sind, ausgebildet. In einer weiteren Ausführungsform, die mit den vorherigen Ausführungsformen kombiniert werden kann, ist der Elektrodenträger dazu ausgebildet, die Messelektrode und die Referenzelektrode in einem definierten Zwischenabstand voneinander zu halten. Dies kann beispielsweise dadurch erreicht werden, dass die Messelektrode und die Referenzelektrode in Trägermaterial eingebettet sind oder indem ein Elektrodenträger Befestigungsmittel an definierten Positionen, beispielsweise die nachfolgend beschriebenen Aussparungen, aufweist.

In einer weiteren Ausführungsform, die mit den vorherigen Ausführungsformen kombiniert werden kann, ist der Elektrodenträger dazu ausgebildet ist, die Messelektrode und/oder die Referenzelektrode ohne Zerstörung des Elektrodenträgers austauschen zu können, insbesondere wobei der Elektrodenträger Aussparungen, insbesondere kreisförmige Aussparungen, zur Aufnahme der Messelektrode und der Referenzelektrode aufweist. Der Elektrodenträger kann einen sich von der Aussparung für die Messelektrode zu der Stirnseite des Elektrodenträgers erstreckenden Kanal aufweisen, insbesondere um eine elektrische Leitung von der Aussparung für die Messelektrode über eine Stirnseite des Elektrodenträgers, insbesondere zur Signalverarbeitungseinheit, abzuführen. Ferner kann der Elektrodenträger einen sich von der Aussparung für die Referenzelektrode zu der Aussparung für die Messelektrode erstreckenden Kanal aufweisen, insbesondere um eine elektrische Leitung von der Referenzelektrode über diesen Kanal und den Kanal zwischen der Aussparung für die Messelektrode und der Stirnseite des Elektrodenträgers, insbesondere zur Signalverarbeitungseinheit, abzuführen. Dadurch können elektrische Leitungen der Messelektrode, der Referenzelektrode und einer ggf. an der Referenzelektrode anliegenden Erdung gemeinsam über die Stirnseite des Elektrodenträgers abgeführt und von dort als Kabel zu der Signalverarbeitungseinheit geleitet werden.

In einer weiteren Ausführungsform, die mit den vorherigen Ausführungsformen kombiniert werden kann, sind die Erdungselektrode und/oder die Referenzelektrode als Elektroden zur einmaligen Verwendung und/oder als ganzflächig leitende Elektroden, insbesondere Elektroden-Pads, ausgebildet. Unter ganzflächig leitenden Elektroden sind vorzugsweise Elektroden zu verstehen, die über eine Klebefläche auf der Haut eines Anwenders angebracht, insbesondere geklebt, werden können, wobei die gesamte Klebefläche als Elektrodenfläche dient, insbesondere elektrisch leitfähig ist. Durch die Verwendung von Einmalelektroden aber auch durch den Einsatz von ganzflächig leitenden Elektroden, kann eine ausreichende Haftung sichergestellt werden, sodass der Kontakt mit der Haut über den Verlauf vieler Stunden über den Tag hinweg aufrechterhalten bleibt. Außerdem ist der Einsatz von Elektroden zur einmaligen Verwendung aus hygienischen Gründen vorteilhaft. Alternativ können für die Referenzelektrode und/oder die Erdungselektrode Dauerelektroden eingesetzt werden, die mit dem Elektrodenträger verklebt und/oder vergossen sein können. Dies geht jedoch mit erhöhten Anforderungen an eine zuverlässige Haftung auf der Haut einher.

In einer weiteren Ausführungsform, die mit den vorherigen Ausführungsformen kombiniert werden kann, sind in und/oder auf dem Elektrodenträger wenigstens abschnittsweise elektrische Leitungen von der Signalverarbeitungseinheit zu der Messelektrode und der Referenzelektrode geführt.

In einer weiteren Ausführungsform, die mit den vorherigen Ausführungsformen kombiniert werden kann, ist die Signalverarbeitungseinheit als von dem Elektrodenträger getrennt positionierbare Einheiten ausgebildet ist, insbesondere wobei die Elektrodeneinheit und die Signalverarbeitungseinheit zur datenübertragungsgemäßen Verbindung über ein Kabel, insbesondere eine isolierte elektrische Leitung, miteinander verbunden sind. Dadurch können gegenüber einer Ausführungsform mit in der Elektrodeneinheit integrierter Signalverarbeitungseinheit Kosten für die Miniaturisierung eingespart werden. In einer alternativen Ausführungsform, die ebenfalls mit den vorherigen Ausführungsformen kombiniert werden kann, ist die Signalverarbeitungseinheit in und/oder auf dem Elektrodenträger ausgebildet, insbesondere eingebettet und/oder verklebt. Vorzugsweise sind dabei eine Energiequelle, insbesondere eine Batterie, ein Prozessor und ein Kommunikationsmittel, insbesondere eine Funkeinheit, der Signalverarbeitungseinheit in und/oder auf dem Elektrodenträger ausgebildet, insbesondere eingebettet und/oder verklebt. Dies geht zwar mit einem erhöhten Kostenaufwand für die Miniaturisierung des Systems einher, birgt aber den Vorteil einer verbesserten Handhabung, da auf ein die Elektrodeneinheit mit der Signalverarbeitungseinheit verbindendes Kabel verzichtet werden kann.

In einer weiteren Ausführungsform, die mit den vorherigen Ausführungsformen kombiniert werden kann, ist die Elektrodeneinheit frei von einer Erdung und/oder weist genau zwei Elektroden, nämlich die Referenzelektrode und die Messelektrode, auf. Gemäß der ersten Variante weist die Elektrodeneinheit keine Erdung auf, wohingegen die Elektrodeneinheit gemäß der Variante Alternative lediglich zwingend keine eigene Elektrode für die Erdung aufweist, jedoch grundsätzlich zulässt, dass eine Erdung in der Messelektrode oder der Referenzelektrode integriert ist. Gemeinsam ist diesen beiden Variante, dass lediglich zwei Elektroden erforderlich sind. Durch den Verzicht auf eine Erdungselektrode weist die Elektrodeneinheit nur zwei relevante Kontaktpunkte, nämlich die der Referenzelektrode und der Messelektrode, auf wodurch auf beliebigen Krümmungen jeweils zwei Kontaktpunkte hergestellt werden können. Dies löst die besondere Herausforderung, welche die individuelle Krümmung des Steißbeins und der Gesäßfalte unterschiedlicher Personen hinsichtlich der Anforderung mit sich bringt, den Kontakt über mehrere Stunden über den Tag verteilt aufrechthalten zu können. Allerdings gehen Ausführungen komplett ohne Erdung mit einer erhöhten Anfälligkeit für Störsignale einher. Daher ist es insbesondere bei Ausführungsformen ohne Erdung besonders vorteilhaft, wenn die Signalverarbeitungseinrichtung dazu ausgebildet ist, das Elektromyographiesignal zu Filtern, insbesondere wie zuvor und nachfolgend beschrieben.

In einer weiteren Ausführungsform, die mit den vorherigen Ausführungsformen kombiniert werden kann, weist die Elektrodeneinheit eine Erdung auf. Durch den Einsatz einer Erdung kann die Anfälligkeit für Störsignale reduziert werden. Die Erdung kann auf der Elektrodenoberfläche der Referenzelektrode ausgebildet sein, wobei vorzugsweise die Elektrodeneinheit genau zwei Elektroden, nämlich die Referenzelektrode und die Messelektrode, aufweist. Mit dieser Ausführungsform kann sowohl die Störanfälligkeit reduziert als auch die Positionierung an beliebigen Krümmungen gewährleistet werden. Auch wenn die Störanfälligkeit bei dieser Ausführungsform durch den Einsatz einer Erdung reduziert wird, ist die Störanfälligkeit größer als bei einer Ausführungsform, bei der die Erdung mittels separater Erdungselektrode erfolgt, die beispielsweise an dem Elektrodenträger angebracht ist. In anderen Worten ist das unmittelbar gemessene Elektromyographiesignal stärker mit Störsignalen behaftet. Um dieses schlechtere Signal besser nutzbar zu machen, kann die Signalverarbeitungseinrichtung dazu ausgebildet sein, das Elektromyographiesignal zu Filtern, insbesondere wie zuvor und nachfolgend beschrieben.

In einer alternativen Ausführungsform weist die Elektrodeneinheit eine zur Messelektrode und Referenzelektrode separate Erdungselektrode auf, in der die Erdung ausgebildet ist. Mit dieser Ausführungsform kann die Anfälligkeit für Störsignale weiter reduziert werden. Auch wenn die Störanfälligkeit bei dieser Ausführungsform durch den Einsatz einer separaten Erdungselektrode weiter reduziert ist, ist die Störanfälligkeit größer als bei einer Ausführungsform, bei der die Erdung beispielsweise beabstandet von dem Elektrodenträger an der Hüfte angebracht ist. In anderen Worten ist das unmittelbar gemessene Elektromyographiesignal stärker mit Störsignalen behaftet. Um dieses schlechtere Signal besser nutzbar zu machen, kann die Signalverarbeitungseinrichtung dazu ausgebildet sein, das Elektromyographiesignal zu Filtern, insbesondere wie zuvor und nachfolgend beschrieben. Jedoch geht dies mit einer geringeren Flexibilität bei der Anbringung an Steißbeine und Gesäßfalten mit unterschiedlichen Krümmungen einher, sodass eine Anpassung der Geometrie der Elektrodeneinheit an die Gesäßfalte erforderlich sein kann. Bei dieser Ausführungsform ist es vorteilhaft, die Messelektrode, die Referenzelektrode und die Erdungselektrode in einer Linie entlang einer ersten Raumachse für die Messung des Elektromyographiesignals anzuordnen. Denn diese Anordnung ist durch den Anwender besonders leicht zu platzieren. Insbesondere erleichtert diese Konfiguration die Anbringung bei Steißbeinen und Gesäßfalten mit geringer Krümmung. Alternativ kann es, insbesondere bei Steißeinen und Gesäßfalten mit großer Krümmung vorteilhaft sein, vorteilhaft, wenn die Elektrodeneinheit derart ausgestaltet ist, dass die Messelektrode, die Referenzelektrode und die Erdungselektrode entlang einer zweiten Raumachse der Elektrodeneinheit abgewinkelt angepasst an die Körperform des Bodens der Gesäßfalte des Anwenders angeordnet sind oder anpassbar ausgestaltet sind. Denn somit kann die Anordnung dem Verlauf der Hautoberfläche in der Gesäßfalte des Anwenders besser folgen und einen optimalen Hautkontakt der Elektroden sicherstellen. Ferner kann es bei dieser Ausführungsform vorteilhaft sein, wenn die Messelektrode und die Referenzelektrode in einem definierten Zwischenabstand voneinander an einem Trägermaterial oder in einem Trägermaterial angeordnet und in der Elektrodeneinheit als Elektroden-Pad gebündelt sind, und durch das Trägermaterial Leitungen von der Signalverarbeitungseinheit zu der Messelektrode, der Referenzelektrode und/oder Erdungselektrode geführt sind. Denn somit ist zum einen ein Anbringen oder ein Tragen in der Gesäßfalte des Anwenders einfach, und zum anderen sind die Leitungen weniger störungsanfällig geführt. Es ist zudem bei dieser Ausführungsform vorteilhaft, wenn die Messelektrode, die Referenzelektrode und die Erdungselektrode kreisförmig ausgelegt sind, denn so wird die Einbettung in das Trägermaterial vereinfacht.

**In** einer weiteren Ausführungsform, die mit den vorherigen Ausführungsformen kombiniert werden kann, weist die Signalverarbeitungseinheit ein Kommunikationsmittel auf das vorzugsweise als Funkeinheit, besonders bevorzugt als ein Bluetooth-Gerät zur funkbasierten Datenübertragung, ausgebildet ist. Bei dieser Ausführungsform ist es vorteilhaft, wenn das Kommunikationsmittel der Signalverarbeitungseinheit als eine standardisierte, insbesondere spielsteuerungs-kompatible, Schnittstelle ausgeführt ist, wobei die standardisierte Schnittstelle vorzugsweise dazu ausgebildet ist, mit einem Endgerät, insbesondere einer Spielsteuerung eines Endgeräts, zu kommunizieren. Denn über diese Schnittstelle kann die Vorrichtung an ein anderes Gerät, wie beispielsweise ein Mobilfunkgerät oder einen Tablet-Computer, angebunden werden. Die gemessenen Werte können durch das Kommunikationsmittel als Daten zur Erzeugung eines Biofeedbacks für ein Training weitergeleitet werden. Somit kann der Anwender der Vorrichtung mittels gamifizierten Übungen zum Training seines Beckenbodens spielerisch mit minimaler mentaler Ermüdung durchführen.

**In** einer weiteren Ausführungsform, die mit den vorherigen Ausführungsformen kombiniert werden kann, ist die Signalverarbeitungseinheit zur Verarbeitung des Elektromyographiesignals ausgelegt, insbesondere zur Umwandlung des Elektromyographiesignals zu einem Steuersignal ausgelegt, insbesondere zu einem Steuersignal, das von einer Spielsteuerung eines Endgeräts als Eingabe verarbeitbar ist.

Ferner betrifft die Erfindung ein System zum spielerischen Trainieren der Beckenbodenmuskulatur, umfassend eine Vorrichtung gemäß dem ersten Aspekt der Erfindung, vorzugsweise mit einer Signalverarbeitungseinheit gemäß dem zweiten Aspekt der Erfindung, ein Endgerät, insbesondere ein mobiles Endgerät, wie ein Mobiltelefon, wobei die Vorrichtung dazu ausgebildet ist, Elektromyographiesignale der Beckenbodenmuskulatur zu messen, das gemessene Elektromyographiesignal in ein Steuersignal umzuwandeln und das Steuersignal an das Endgerät zu senden, insbesondere wobei das Endgerät dazu ausgebildet ist, das Signal als optisches Biofeedback anzuzeigen, das sich vorzugsweise ändert, wenn ein Anwenders die Spannung in der Beckenbodenmuskulatur ändert. Vorzugsweise sind die Vorrichtung und das Endgerät als voneinander getrennte Vorrichtungen ausgebildet, wobei das Steuersignal vorzugsweise über eine Drahtlosverbindung an das Endgerät gesendet werden kann.

**In** einer Ausführungsform weist das Endgerät ein Spiel oder mehrere auf und ist dazu ausgelegt, das Spiel oder die Spiele über das Steuersignal zu steuern, insbesondere derart, dass das Spiel oder die Spiele über Anspannung der Beckenbodenmuskulatur gesteuert werden kann.

**In** einer Ausführungsform des zweiten Aspekts der Erfindung ist die Signalverarbeitungseinheit zur Auswertung einer Aktivität einer Beckenbodenmuskulatur über ein Elektromyographiesignal dazu ausgebildet, die Kalibrierung automatisch durchzuführen, indem ein Ruhewert für das Elektromyographiesignal der nicht kontrahierten Beckenbodenmuskulatur statistisch erfasst wird, und die Ermittlung der Stärke einer Kontraktion der Beckenbodenmuskulatur anhand einer Referenzierung zu dem Ruhewert erfolgt. Auf diese Weise kann eine Anpassung des Trainings individuell auf einen Anwender umgesetzt werden. Vor der vorliegenden Erfindung war es üblich, dass der Patient bei Elektromyographie basierten Trainingsgeräten minimale und maximale Kontraktionen der Beckenbodenmuskulatur durchführt, und anhand dieser beiden Extremwerte das System kalibriert wird. Dies war der etablierte Weg, um das Messsignal auf die individuellen Eigenschaften des Patienten abzustimmen. Die Idee, stattdessen eine automatische Kalibrierung anhand eines statistisch erfassten Ruhewerts durchzuführen, also ohne, dass der Patient aktiv Kontraktionen durchführen muss, entspringt folgender Überlegung. Viele Patienten mit Beckenbodenbeschwerden können nicht oder nur unter Schmerzen maximale Kontraktionen durchführen. Eine Kalibrierungsmethode, die diese belastenden Manöver erfordert, ist für diese Patienten ungeeignet. Eine Erkenntnis der vorliegenden Erfindung war es, dass man den Ruhewert (also das Signal bei entspannter Muskulatur) als Kalibrierungsreferenz nutzen kann. Durch die Ermittlung der Stärke einer Kontraktion der Beckenbodenmuskulatur anhand einer Referenzierung zu dem Ruhewert wird ermöglicht, dass Patienten mit Beckenbodenbeschwerden bereits bei leichter oder mittlerer Muskelanstrengung eine aussagekräftige Rückmeldung erhalten, ohne sich bis zur maximalen Anspannung anstrengen zu müssen. Dies macht das Training angenehmer, sicherer und vor allem für eine breitere Patientengruppe, einschließlich Patienten mit schwacher oder ungünstig lokalisierbarer Muskulatur, zugänglich.

**In** einer weiteren Ausführungsform des zweiten Aspekts der Erfindung, die mit der vorherigen Ausführungsform kombiniert werden kann, ist die Signalverarbeitungseinheit dazu ausgebildet, bei der Ermittlung einer Kontraktion der Beckenbodenmuskulatur eine Stärke der Kontraktion mittels maschineller Klassifikation in diskrete Werte für Intensitäten abzubilden. Durch eine Klassifikation kann ein Klassifikator erlernt werden und in der Signalverarbeitungseinheit eingesetzt werden, die dann mit einer hohen Genauigkeit unterschiedlich starke Muskelkontraktionen verschiedene Intensitätsstufen zuordnet.

**In** einer weiteren Ausführungsform des zweiten Aspekts der Erfindung, die mit den vorherigen Ausführungsformen kombiniert werden kann, ist die Signalverarbeitungseinheit dazu ausgebildet, dass die Rückmeldung der Kontraktion der Beckenbodenmuskulatur als Biofeedback über eine Schnittstelle zur Steuerung von Videospielen erfolgt. Dadurch können die Vorteile einer Gamifizierung für das Training des Beckenbodens genutzt werden.

Ausführungsbeispiele der Erfindung sind in den Zeichnungen dargestellt und in der nachfolgenden Beschreibung näher erläutert. Es zeigen
- Figur 1: eine schematische Darstellung eines Anwenders mit Positionierung einer ersten Ausführungsform der erfindungsgemäßen Vorrichtung zum Training der Beckenbodenmuskulatur,
- Figur 2: eine perspektivische Ansicht auf einen Bereich der Vorrichtung aus Figur 1,
- Figur 3: eine schematische Darstellung eines Anwenders mit Positionierung einer zweiten Ausführungsform der erfindungsgemäßen Vorrichtung zum Training der Beckenbodenmuskulatur,
- Figur4: eine Aufsicht auf einen Bereich der der Vorrichtung aus Figur 3,
- Figur 5: eine perspektivische Ansicht auf einen Bereich einer dritten Ausführungsform der erfindungsgemäßen Vorrichtung,
- Figur 6: die Funktionen der erfindungsgemäßen Signalverarbeitungseinheit.

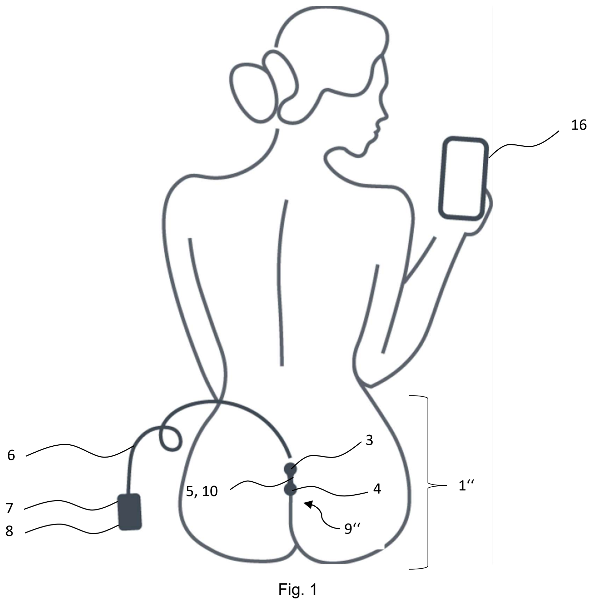

Figur 1 zeigt eine erste Ausführungsform der erfindungsgemäßen Vorrichtung 1". Die Vorrichtung 1" weist eine Elektrodeneinheit 9" und eine Signalverarbeitungseinheit 7 auf, die über eine isolierte elektrische Leitung 6 (Kabel) miteinander verbunden sind. Die Signalverarbeitungseinheit 7 weist ein Kommunikationsmittel 8 zur Kommunikation mit einem als Mobiltelefon 16 ausgebildeten Endgerät auf.

Die Elektrodeneinheit 9" weist genau zwei Elektroden auf, nämlich eine Messelektrode 4 und eine Referenzelektrode 3, wobei die Referenzelektrode 3 vorzugsweise eine Erdung aufweist. **In** der dargestellten Ausführungsform sind die Messelektrode 4 und die Referenzelektrode 3 als Elektroden zur einmaligen Verwendung (Einmalelektroden) ausgebildet.

Ferner weist die Elektrodeneinheit 9" einen Elektrodenträger 10 auf, an dem die Messelektrode 4 und die Referenzelektrode 3 befestigt sind, um die Messelektrode und die Referenzelektrode 3 in einem vordefinierten Abstand zueinander zu halten. Der Elektrodenträger 10 kann aus einem Trägermaterial 5, insbesondere aus hautverträglichem Material, gebildet sein.

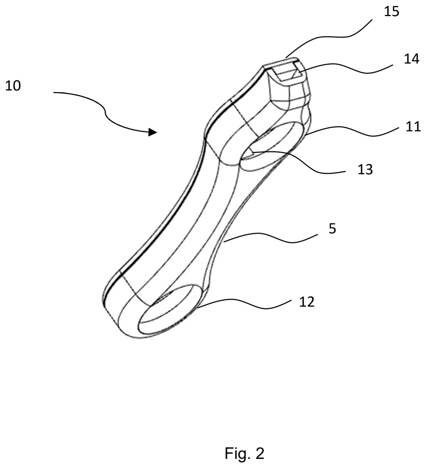

Figur 2 zeigt eine perspektivische Ansicht des Elektrodenträgers 10. Dieser weist eine kreisförmige Aussparung 11 zur Aufnahme der Messelektrode 4 und eine kreisförmige Aussparung 12 zur Aufnahme der Referenzelektrode 3 auf. Ferner weist der Elektrodenträger 10 einen sich von der Aussparung 11 für die Messelektrode zu der Stirnseite des Elektrodenträgers 10 erstreckenden Kanal 13 auf, insbesondere um eine elektrische Leitung von der Aussparung 11 für die Messelektrode 4 über die Stirnseite 15 des Elektrodenträgers 10 abzuführen. Ferner weist der Elektrodenträger 10 einen sich von der Aussparung 12 für die Referenzelektrode 3 zu der Aussparung 11 für die Messelektrode 4 erstreckenden Kanal 14 auf, insbesondere um eine elektrische Leitung von der Referenzelektrode 3 über die Aussparung 11 für die Messelektrode 4 und dem Kanal 13 über die Stirnseite 15 des Elektrodenträgers 10 abzuführen. Dadurch können elektrische Leitungen der Messelektrode 4, der Referenzelektrode 3 und der ggf. an der Referenzelektrode 3 anliegenden Erdung, gemeinsam über die Stirnseite 15 des Elektrodenträgers abgeführt und von dort als Kabel 6 zu der Signalverarbeitungseinheit 7 geleitet werden.

Wie in Figur 2 dargestellt, ist der Elektrodenträger 10 vorzugsweise länglich, insbesondere knochenförmig ausgebildet, wodurch eine korrekte Positionierung des Elektrodenträgers 10 im Bereich zwischen Steißbein und Anus erleichtert wird.

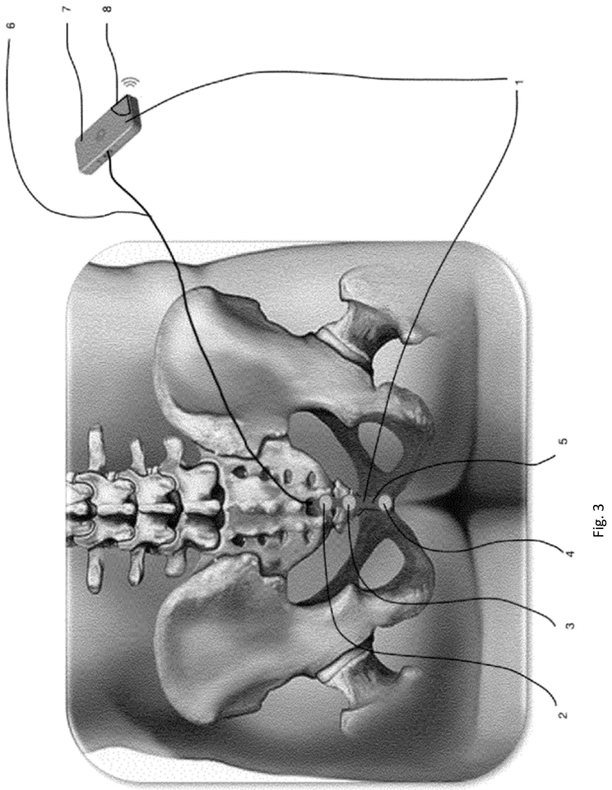

**In** den nachfolgend beschriebenen Figuren 3 bis 6 werden zum ersten Ausführungsbeispiel der Vorrichtung 1" und der Elektrodeneinheit 9" alternative Ausführungsbeispiele zwei und drei beschrieben und mit den Bezugszeichen 1 und 1' bzw. 9 und 9' versehen. Diese Ausführungsbeispiele unterscheiden sich im Wesentlichen dadurch von der ersten Ausführungsbeispiele, dass eine separate Erdungselektrode 2 eingesetzt wird. Dies wird am Beispiel von Elektrodeneinheiten gezeigt, in denen die Elektroden in das Trägermaterial eingebettet sind, diese also keine Einmalelektroden sind. Es sei jedoch klar, dass sämtliche im Zusammenhang mit den Ausführungsbeispielen zwei und drei beschriebenen Ausführungen und Details auch bei Ausführungsformen ohne Erdungselektrode und/oder bei Ausführungsformen mit Einmalelektroden ausgeführt sein können und dass die zuvor im Zusammenhang mit dem ersten Ausführungsbeispiel beschriebenen Ausführungen und Details auch bei Ausführungsformen mit dauerhaft genutzten, insbesondere eingebetteten, Elektroden und/oder bei Ausführungsformen mit separater Erdungselektrode eingesetzt werden können und umgekehrt.

Figur 3 zeigt eine schematische Darstellung einer Anatomie eines Anwenders in Rückenansicht, der eine zweiten Ausführungsform der erfindungsgemäßen Vorrichtung 1 zur Auswertung einer Muskelaktivität seiner Beckenbodenmuskulatur mittels einer Messung eines Elektromyographiesignals der Muskelaktivität benutzt.

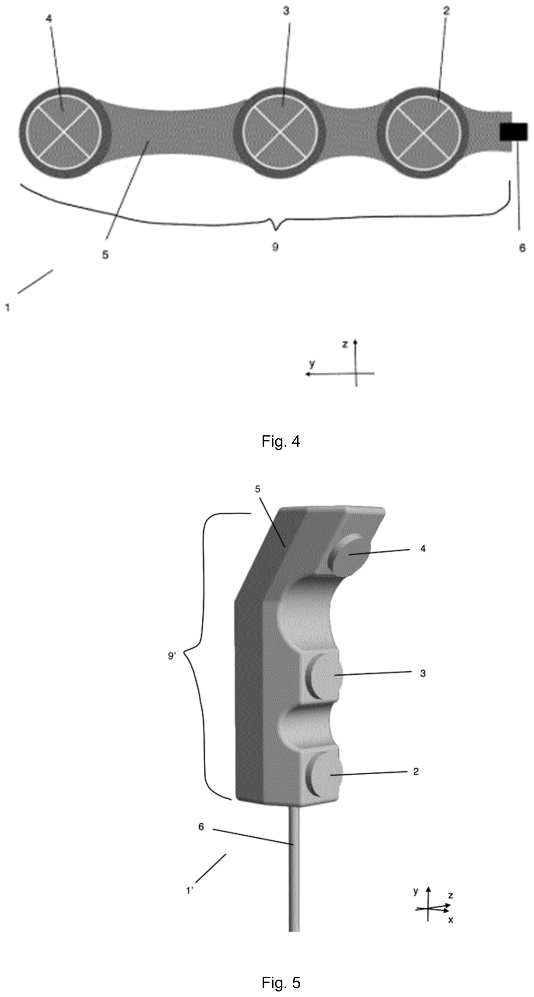

Figur 4 zeigt eine Aufsicht auf eine Elektrodeneinheit 9 der erfindungsgemäßen Vorrichtung 1 gemäß der zweiten Ausführungsform.

Die Vorrichtung 1 aus Figur 3 weist eine Signalverarbeitungseinheit 7 mit einem Kommunikationsmittel 8, eine Leitung 6, ein Trägermaterial 5 und eine Elektrodeneinheit 9 mit einer Messelektrode 4, einer Referenzelektrode 3 und einer Erdungselektrode 2 auf.

Die Vorrichtung 1 ist so ausgelegt, dass das Trägermaterial 5 mit der Messelektrode 4, der Referenzelektrode 3 und der Erdungselektrode 2 die Elektrodeneinheit 9, ein Elektroden-Pad, bildet, das extrakorporal am Steißbein des Anwenders angebracht werden kann.

Die Elektrodeneinheit 9 und damit die Messelektrode 4, die Referenzelektrode 3 und die Erdungselektrode 2 sind mit der Signalverarbeitungseinheit 7 über die Leitung 6 verbunden. Die Elektrodeneinheit 9 ist so ausgelegt, dass das Elektromyographiesignal gemessen werden und über die angebundene Signalverarbeitungseinheit 7 verarbeitet werden kann.

Die Messelektrode 4, die Referenzelektrode 3 und die Erdungselektrode 2 befinden sich in einem definierten Zwischenabstand voneinander, welcher durch das umgossene Trägermaterial 5, beispielsweise aus Nylon, gleich gehalten wird. Der Abstand zwischen der Messelektrode 4, der Referenzelektrode 3 und der Erdungselektrode 2 beträgt beispielsweise 40-60 mm.

Die Messelektrode 4, die Referenzelektrode 3 und die Erdungselektrode 2 bilden eine Linie entlang einer ersten Raumachse (als y- Achse dargestellt).

**In** einer vierten Ausführungsform sind die Messelektrode 4, die Referenzelektrode 3 und die Erdungselektrode 2 in Form eines Dreiecks angeordnet.

Die Leitung 6 zwischen der Signalverarbeitungseinheit 7 und der Messelektrode 4, der Referenzelektrode 3 und der Erdungselektrode 2 wird durch das Trägermaterial 5 geführt. Die Leitung 6 ist beispielsweise ein ummanteltes Kabel.

Die Messelektrode 4, die Referenzelektrode 3 und die Erdungselektrode 2 sind beispielsweise jeweils kreisförmig ausgelegt, so dass deren Durchmesser beispielsweise 5-10 mm beträgt, und sind beispielsweise als Ag/AgCl-Elektroden ausgeführt.

Das Kommunikationsmittel 8 der Signalverarbeitungseinheit 7 ist als eine standardisierte Geräteschnittstelle, beispielsweise eine Spielsteuerung (Gamecontroller)-kompatible Schnittstelle, ausgelegt. Beispielsweise ist das Kommunikationsmittel 8 als ein Bluetooth-Gerät zur funkbasierten Datenübertragung umgesetzt, die es ermöglicht, mit anderen schnittstellenkompatiblen Geräten zu kommunizieren. Auf diese Weise kann die Signalverarbeitungseinheit 7 schnittstellenkompatiblen Geräten Daten liefern, die als Steuerungsdaten einer Spielsteuerung (eines Gamecontrollers) entsprechend verarbeitet werden. Die Signalverarbeitungseinheit 7 kann bei den schnittstellenkompatiblen Geräten so beispielsweise Elemente in Computerspielen steuern. Der Anwender ist auf diese Weise in der Lage, über gezielte Muskelkontraktionen der Beckenbodenmuskulatur Computerspiele zu steuern.

Figur 5 zeigt eine perspektivische Darstellung einer Elektrodeneinheit 9' in einer dritten Ausführungsform der erfindungsgemäßen Vorrichtung 1' zur Auswertung der Muskelaktivität der Beckenbodenmuskulatur des menschlichen Anwenders.

Bei der dritten Ausführungsform der Vorrichtung 1' ist die Elektrodeneinheit 9' so ausgelegt, dass die Messelektrode 4 entlang einer zweiten Raumachse (als x-Achse dargestellt) abgewinkelt angepasst an die Körperform der Gesäßfalte des Anwenders angeordnet ist.

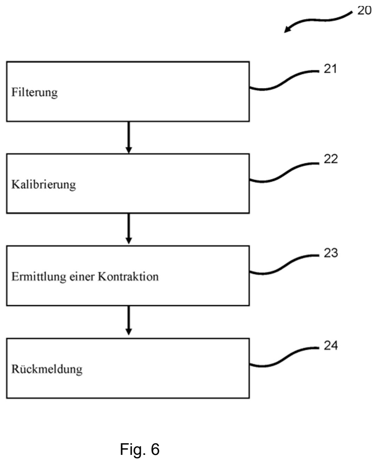

Figur 6 zeigt die Funktionen 20 einer erfindungsgemäßen Signalverarbeitungseinheit 7 zur Auswertung einer Muskelaktivität eines Beckenbodenmuskels über ein Elektromyographiesignal einer extrakorporal auf das Steißbein angelegten Elektrodeneinheit, die beispielsweise eine Messelektrode, eine Referenzelektrode und eine Erdungselektrode aufweist. Die Signalverarbeitungseinheit 7 ist dazu ausgelegt, vier Funktionen 21, 22, 23, 24 zu erfüllen

Gemäß der ersten Funktion 21 ist die Signalverarbeitungseinheit 7 dazu ausgebildet, Messwerte des Elektromyographiesignals zu filtern. Die Signalverarbeitungseinheit 7 kann dazu ausgebildet sein, die Filterung durch entsprechende Methoden und Filter aus der Signalverarbeitung, wie beispielsweise Hochpass-, Tiefpass- und/oder Bandpassfilter, durchzuführen.

Gemäß der zweiten Funktion 22 ist die Signalverarbeitungseinheit 7 dazu ausgebildet, das gefilterte Elektromyographiesignal zu kalibrieren. Die Signalverarbeitungseinheit 7 kann dazu ausgebildet sein, die Kalibrierung automatisch durch eine statistische Erfassung von Messwerten, die einen Ruhewert des Elektromyographiesignals der nicht-kontrahierten Beckenbodenmuskulatur darstellen, sowie einer Ermittlung einer Kontraktion der Beckenbodenmuskulatur mittels einer Referenzierung weiterer, folgender Messwerte zu dem Ruhewert durchzuführen.

Gemäß der dritten Funktion 23 ist die Signalverarbeitungseinheit 7 dazu ausgebildet, aus dem kalibrierten Elektromyographiesignal eine Kontraktion der Beckenbodenmuskulatur zu ermitteln. Die Signalverarbeitungseinheit 7 kann dazu ausgebildet sein, die Ermittlung der Kontraktion der Beckenbodenmuskulatur mittels maschineller Klassifikation und der Abbildung des kalibrierten Messwerts in diskrete Werte für Intensitäten durchzuführen. Beispielsweise kann die Signalverarbeitungseinheit 7 dazu ausgebildet sein, für die Klassifikation die lineare Diskriminantenanalyse, Support-Vector-Maschinen, Entscheidungsbäume, Bayes-Klassifikationen und/oder neuronale Netze zu verwenden. Diskrete Werte für die Intensitäten sind beispielsweise 25%-Schritte zwischen dem Ruhewert und der maximalen gemessenen Muskelkontraktion.

Gemäß der vierten Funktion 24 ist die Signalverarbeitungseinheit 7 dazu ausgebildet, die ermittelte Kontraktion an einen Anwender über eine Ausgabe zu melden, beispielsweise als sogenanntes Biofeedback. Dafür kann die Signalverarbeitungseinheit 7 dazu ausgebildet sein, das verarbeitete und normierte Elektromyographiesignal zu einem Steuersignal umzuwandeln und kabellos über beispielsweise Bluetooth **LE** anderen Geräten zur Verfügung zu stellen. Ferner kann die Signalverarbeitungseinheit 7 dazu ausgebildet sein, das Biofeedback als Steuersignal über eine Spielsteuerung (Gamecontroller)-kompatible Schnittstelle an andere Geräte weiterzuleiten, die das Steuersignal als Eingabe einer Spielsteuerung (eines Gamecontrollers) interpretieren und verarbeiten.

## Patentansprüche

1. Vorrichtung (1, 1', 1") zur Auswertung einer Muskelaktivität einer Beckenbodenmuskulatur eines Beckenbodens, wobei die Vorrichtung (1) eine Signalverarbeitungseinheit (7) und eine extrakorporale Elektrodeneinheit (9, 9', 9") aufweist,
- wobei die Elektrodeneinheit (9, 9', 9") eine Messelektrode (4) und eine Referenzelektrode (3) aufweist,
- wobei die Elektrodeneinheit (9, 9', 9") ausgelegt ist, ein Elektromyographiesignal zu messen.

2. Vorrichtung (1, 1', 1") nach Anspruch 1,
wobei die Messelektrode (4) und/oder die Referenzelektrode (3) Elektroden zur einmaligen Verwendung und/oder ganzflächig leitende Elektroden, insbesondere Elektroden-Pads, sind.

3. Vorrichtung nach Ansprüche 1 oder 2,
wobei die Elektrodeneinheit frei von einer Erdung ist und/oder genau zwei Elektroden, nämlich die Referenzelektrode (4) und die Messelektrode (3), aufweist.

4. Vorrichtung (1, 1', 1") nach Anspruch 1 oder 2,
wobei die Elektrodeneinheit (9, 9', 9") eine Erdung aufweist.

5. Vorrichtung (1") nach Anspruche 4,
wobei die Erdung auf der Elektrodenoberfläche der Referenzelektrode (3) ausgebildet ist und/oder wobei die Elektrodeneinheit (9") genau zwei Elektroden, nämlich die Referenzelektrode (3) und die Messelektrode (4), aufweist.

6. Vorrichtung (1, 1', 1") nach einem der Ansprüche 1 bis 5,
wobei die Elektrodeneinheit (9, 9', 9") datenübertragungsgemäß, insbesondere mittels einer elektrischen Leitung, mit der Signalverarbeitungseinheit (7) verbunden ist, insbesondere um das gemessene Elektromyographiesignal von der Elektrodeneinheit an die Signalverarbeitungseinheit (7) zu übertragen.

7. Vorrichtung (1, 1', 1") nach einem der Ansprüche 1 bis 6,
wobei die Elektrodeneinheit (9, 9', 9") einen Elektrodenträger (10) aufweist in und/oder auf dem die Messelektrode (4) und die Referenzelektrode (3) angeordnet, insbesondere befestigt, sind, insbesondere wobei der Elektrodenträger (10) dazu ausgebildet ist, die Messelektrode (4) und die Referenzelektrode (3) in einem definierten Zwischenabstand voneinander zu halten.

8. Vorrichtung (1, 1', 1") nach Anspruch 7,
wobei der Elektrodenträger (10) dazu ausgebildet ist, dass die Messelektrode (4) und/oder die Referenzelektrode (3) ohne Zerstörung des Elektrodenträgers (10) ausgetauscht werden können, insbesondere wobei der Elektrodenträger (10) Aussparungen, insbesondere kreisförmige Aussparungen zur Aufnahme der Messelektrode (4) und der Referenzelektrode (3), aufweist.

9. Vorrichtung (1, 1', 1") nach einem der Ansprüche 7 bis 8,
wobei in und/oder auf dem Elektrodenträger (10) wenigstens abschnittsweise elektrische Leitungen (6) von der Signalverarbeitungseinheit (7) zu der Messelektrode (4) und der Referenzelektrode (3) geführt sind.

10. Vorrichtung (1, 1', 1") nach einem der Ansprüche 7 bis 9,
wobei die Signalverarbeitungseinheit (7) als von dem Elektrodenträger getrennt positionierbare Einheiten ausgebildet ist, insbesondere wobei die Elektrodeneinheit (9, 9', 9") und die Signalverarbeitungseinheit (7) zur datenübertragungsgemäßen Verbindung über ein Kabel (6), insbesondere eine isolierte elektrische Leitung, miteinander verbunden sind.

11. Vorrichtung (1, 1', 1") nach einem der Ansprüche 7 bis 9,
wobei die Signalverarbeitungseinheit (7) in und/oder auf dem Elektrodenträger (10) ausgebildet, insbesondere eingebettet und/oder verklebt, ist.

12. Vorrichtung (1, 1', 1") nach einem der Ansprüche 1 bis 11,
wobei die Signalverarbeitungseinheit (7) ein Kommunikationsmittel (8), das vorzugsweise als Funkeinheit ausgebildet ist, aufweist, insbesondere wobei das Kommunikationsmittel (8) der Signalverarbeitungseinheit (7) als eine standardisierte, insbesondere spielsteuerungs-kompatible, Schnittstelle ausgeführt ist, insbesondere wobei die standardisierte Schnittstelle dazu ausgebildet ist, mit einem Endgerät, insbesondere einer Spielsteuerung eines Endgeräts, zu kommunizieren, und/oder wobei die Signalverarbeitungseinheit (7) zur Verarbeitung des Elektromyographiesignals ausgelegt ist, insbesondere zur Umwandlung des Elektromyographiesignals zu einem Steuersignal ausgelegt ist, insbesondere zu einem Steuersignal, das von einer Spielsteuerung eines Endgeräts als Eingabe verarbeitbar ist.

13. Vorrichtung (1, 1', 1") nach einem der vorstehenden Ansprüche, wobei die Signalverarbeitungseinheit (7) dazu ausgebildet ist,
- das Elektromyographiesignal zu Filtern (21);
- das gefilterten Elektromyographiesignal zu kalibrieren (22);
- eine Kontraktion und/oder eine Kontraktionsstärke der Beckenbodenmuskulatur anhand des gefilterten und kalibrierten Elektromyographiesignals zu ermitteln (23); und
- die Kontraktion zurückzumelden (24), insbesondere in Form eines Biofeedbacks an einen Anwender, insbesondere an die Person deren Beckenbodenaktivität ausgewertet wird.

14. Vorrichtung (1, 1', 1") nach Anspruch 13,
wobei die Signalverarbeitungseinheit (7) dazu ausgelegt ist, für die Kalibrierung (22) automatisch einen Ruhewert des Elektromyographiesignals der nicht kontrahierten Beckenbodenmuskulatur statistisch zu erfassen, und die Ermittlung (23) der Kontraktion und/oder der Kontraktionsstärke anhand einer Referenzierung zu dem Ruhewert durchzuführen, insbesondere wobei die Signalverarbeitungseinheit (7) dazu ausgebildet ist, im Rahmen der Ermittlung (23) der Kontraktion der Beckenbodenmuskulatur mittels maschineller Klassifikation eine Stärke der Kontraktion in diskrete Werte für Intensitäten abzubilden, und/oder wobei die Signalverarbeitungseinheit (7) dazu ausgebildet ist, die Rückmeldung (24) der Kontraktion der Beckenbodenmuskulatur über Biofeedback an eine standardisierte Schnittstelle durchzuführen.

15. System zum spielerischen Trainieren der Beckenbodenmuskulatur, umfassend
- eine Vorrichtung (1, 1', 1") nach einem der Ansprüche 1 bis 14,
- ein Endgerät, insbesondere ein Mobiltelefon,
wobei die Vorrichtung (1, 1', 1") dazu ausgebildet ist, Elektromyographiesignale der Beckenbodenmuskulatur zu messen, das gemessene Elektromyographiesignal in ein Steuersignal umzuwandeln und das Steuersignal an das Endgerät zu senden, insbesondere wobei das Endgerät dazu ausgebildet ist, das Signal als optisches Biofeedback anzuzeigen, das sich vorzugsweise ändert, wenn ein Anwender die Spannung in der Beckenbodenmuskulatur ändert, insbesondere wobei das Endgerät ein Spiel aufweist und dazu ausgelegt ist, das Spiel über das Steuersignal zu steuern, insbesondere derart, dass das Spiel über Anspannung der Beckenbodenmuskulatur gesteuert werden kann.
